# EUROPEAN PATENT SPECIFICATION

(11) **EP 3 144 329 B1**
(45) Date of publication and mention of the grant of the patent: **28.02.2018**
(21) Application number: 15792655.1
(22) Date of filing: 14.05.2015
(51) Int. Cl.: C08F 297/02, C08F 212/08, C08F 210/02, C08F 212/36, C08F 6/02, A61L 29/00, A61L 31/00, H01L 31/048, C08F 4/6592, C08J 3/24

(54) **CROSS-COPOLYMER AND METHOD FOR PRODUCING SAME**
CROSS-COPOLYMER UND VERFAHREN ZUR HERSTELLUNG DAVON
COPOLYMÈRE RÉTICULÉ ET SON PROCÉDÉ DE PRODUCTION

(30) Priority: 15.05.2014 JP 2014101130
(43) Date of publication of application: 22.03.2017
(73) Proprietor: Denka Company Limited, Tokyo 103-8338 (JP)
(72) Inventor: ARAI, Toru, Tokyo 103-8338 (JP); TSUKAMOTO, Ayumu, Machida-city Tokyo 194-8560 (JP); SASAKI, Eri, Machida-city Tokyo 194-8560 (JP)
(74) Representative: Gulde & Partner
(86) International application number: PCT/JP2015/063873
(87) International publication number: WO 2015/174485

(56) References cited:
- EP-A1- 1 170 313
- WO-A1-2013/018839
- JP-A- 2010 043 232
- JP-A- 2011 213 843
- JP-A- 2013 202 133
- US-A1- 2009 263 604

## Description

### Technical Field

The present invention relates to a cross-copolymer in which a catalyst component used when the cross-copolymer is synthesized remains in a reduced amount and a method for producing the cross-copolymer.

### Background Art

A resin of a cross-copolymer which is a block copolymer having a branched structure containing an olefin-(aromatic vinyl compound)-(aromatic polyene) copolymer chain and a polystyrene chain has been disclosed (Patent Literatures 1, 2, and 5).

This resin is a flexible, heat-resistant elastomer. Meanwhile, this resin can be produced through: a coordination polymerization step of obtaining an olefin-(aromatic vinyl compound)-(aromatic polyene) copolymer as a macromonomer; and a crossing step of producing a polystyrene chain in the presence of this macromonomer and linking the polystyrene chain to the macromonomer.

In addition, a method for removing a lithium catalyst component from a styrene-butadiene block copolymer, which can be produced by living anionic polymerization, or a hydrogenated product thereof has also been disclosed. Example of the method known include: a method comprising washing a lithium component away by adding acidic water to a polymer solution obtained by anionic polymerization; and a method for removing a lithium component from a polymer solution, in which an alcohol and/or an acid have been added to polymerization liquid containing a conjugated diene-based polymer, by making the polymer solution and water together pass through a specific rotary dispersing device, so that the mixture is subject to high shearing dispersion (Patent Literatures 3 and 4).

### Citation List

### Patent Literature

[Patent Literature 1] WO00/37517
[Patent Literature 2] WO2007/139116
[Patent Literature 3] JP11-335432A
[Patent Literature 4] JP06-136034A
[Patent Literature 5] WO 2013/018839 A1

### Summary of Invention

### Technical Problem

Unfortunately, the conventional technologies as described in the above literatures have had room for improvement regarding the following points.

Patent Literatures 1 and 2 describe that aluminum, which is a promoter component used for a metallocene catalyst, used in a coordination polymerization step and lithium, which is a component of an anionic polymerization initiator, used in a crossing step remain in a significant amount in a cross-copolymer. In addition, these residual catalyst components may cause yellowish discoloration of a resin because of an interaction with a stabilizer added to the resin.

Further, when the content of the catalyst remaining in the resin is large, there remain concerns from the viewpoint of improving safety and performance when the resin is used for medical materials and specific electronic and electric materials.

Patent Literature 3 only describes that a polymer solution containing a specific block copolymer and a residual catalyst is washed with acidic water, but is silent about an industrially advantageous washing method.

Patent Literature 4 describes an industrially advantageous lithium removal method comprising washing a conjugated diene-based polymer-containing polymer solution with water under a specific high shearing condition. In addition, Patent Literature 4 also describes that when an acid and an alcohol are beforehand added to an organic layer, the lithium removal efficiency increases.

However, it cannot be said that in these known methods, their removal efficiency is sufficient. Consequently, a more efficient removal method has been sought.

The present invention has been made in view of the above situations, and the purpose of the present invention is to provide: a cross-copolymer in which a residual catalyst component remains in a reduced amount and which has improved transparency, applicability to medical materials, and yellowish discoloration resistance; and a method for producing the cross-copolymer.

### Solution to Problem

An aspect of the present invention provides a cross-copolymer which is produced through a coordination polymerization step of carrying out copolymerization of an olefin monomer, an aromatic vinyl compound monomer and an aromatic polyene using a single-site coordination polymerization catalyst to synthesize an olefin-(aromatic vinyl compound)-(aromatic polyene) copolymer and a subsequent anionic polymerization step of carrying out polymerization in a co-presence of the olefin-(aromatic vinyl compound)-(aromatic polyene) copolymer and an aromatic vinyl compound monomer using an anionic polymerization initiator,
wherein a total mass of aluminum and lithium, which are residual catalyst components, contained in the cross-copolymer is 200 ppm or less.

In addition, another aspect of the present invention provides a method for producing a cross-copolymer, comprising the steps of:
obtaining a polymer solution containing a cross-copolymer;
preparing liquid containing the polymer solution, water, and an organic acid and subjecting the liquid to emulsification/dispersion, and thereafter;
separating and removing the water from the polymer solution; and
collecting the cross-copolymer from the polymer solution,
wherein the cross-copolymer is produced through a coordination polymerization step of carrying out copolymerization of an olefin monomer, an aromatic vinyl compound monomer and an aromatic polyene using a single-site coordination polymerization catalyst to synthesize an olefin-(aromatic vinyl compound)-(aromatic polyene) copolymer and a subsequent anionic polymerization step of carrying out polymerization in a co-presence of the olefin-(aromatic vinyl compound)-(aromatic polyene) copolymer and an aromatic vinyl compound monomer using an anionic polymerization initiator, and
wherein the organic acid has a pKa of from 1 to 7 and
solubility of the organic acid is 5 g or more per 100 g of water at 20°C.

### Advantages

The present invention can provide a cross-copolymer in which a residual catalyst component remains in a reduced amount that is a prescribed level or less. In this cross-copolymer, the residual catalyst component remains in a reduced amount. Accordingly, the cross-copolymer is very applicable to medical materials. In addition, yellowish discoloration caused by addition of an antioxidant is suppressed and its transparency is enhanced as well.

Further, the present invention can provide a method for producing a cross-copolymer in which a residual catalyst component remains in a reduced amount because a metal catalyst can be highly efficiently removed from a polymer solution in an industrially advantageous manner.

### Description of Embodiments

Hereinafter, embodiments of the present invention will be described in detail.

An embodiment of the present invention provides a cross-copolymer which is produced through a coordination polymerization step of carrying out copolymerization of an olefin monomer, an aromatic vinyl compound monomer and an aromatic polyene using a single-site coordination polymerization catalyst to synthesize an olefin-(aromatic vinyl compound)-(aromatic polyene) copolymer and a subsequent anionic polymerization step of carrying out polymerization in the co-presence of the olefin-(aromatic vinyl compound)-(aromatic polyene) copolymer and an aromatic vinyl compound monomer using an anionic polymerization initiator,
wherein the total mass of aluminum and lithium, which are residual catalyst components, contained in the cross-copolymer is 200 ppm or less.

Conventional cross-copolymers and manufacturing methods thereof are disclosed in, for example, WO2000/37517, US Patent No. 6559234, or WO2007/139116.

A catalyst containing a transition metal compound and alumoxane (e.g., methylaluminoxane (or referred to as methyl alumoxane or MAO)), which is a promoter, may be used as a single site coordination polymerization catalyst used in the coordination polymerization. In this case, such a single site coordination polymerization catalyst is characterized by its high polymerization activity and stability. However, a relatively large amount of the catalyst has to be used. Accordingly, a relatively large amount of a residual catalyst component, in particular, aluminum is included in a final polymer.

Further, the alumoxane used in the coordination polymerization reacts with an anionic polymerization initiator used in the subsequent anionic polymerization step. Thus, an additional portion of the anionic polymerization initiator that will be consumed during the reaction with the alumoxane has to be added. That is, when alumoxane is used in the coordination polymerization, a relatively large amount of the anionic polymerization initiator has to be used during the anionic polymerization step.

Note that because a lithium compound (e.g., butyl lithium) is used as the anionic polymerization initiator, a relatively large amount of lithium is unfortunately included in the final polymer.

In order to solve the problems, the present inventors have tried various polymerization methods and methods for removing a catalyst component. In the cross-copolymer as obtained through the above coordination polymerization step and anionic polymerization step, however, a significant amount of the catalyst components remains. Even when the conventional methods for removing a catalyst component was used, the amount of the components removed was relative small. Thus, a large amount of the catalyst components remained in the cross-copolymer.

Nevertheless, the present inventors have conducted intensive research. As a result, the present inventors have found that when the cross-copolymer-containing polymer solution is processed with an emulsifying disperser, the catalyst components can be efficiently removed from the polymer solution.

Then, the present inventors have successfully produced a cross-copolymer through the above coordination polymerization step and the subsequent anionic polymerization step, in which cross-copolymer the residual catalyst components remain in such a reduced amount that the total mass of aluminum and lithium, which are the catalyst components, contained in the cross-copolymer is 200 ppm or less.

Preferably, the total mass of aluminum and lithium, which are the residual catalyst components according to this embodiment, contained in the cross-copolymer is 100 ppm or less. More preferably, the total mass is 50 ppm or less.

In the cross-copolymer according to this embodiment, the residual catalyst components remain in a reduced amount. Accordingly, the cross-copolymer is characterized by having excellent applicability to, for example, medical materials. Examples of suitable applications include materials for medical tubes and medical bags. A standard for medical materials is defined by a range of change in the pH of water after extraction using boiling water. This means that there is a case in which when the amount of a catalyst component extracted is large, the cross-copolymer may not be used for the medical material.
The cross-copolymer according to this embodiment can satisfy the range of the standard for medical materials without further removing the catalyst components. In medical use, the change in pH and the amount of the catalyst components extracted should be as small as possible. Hence, the cross-copolymer according to this embodiment can be suitably used for medical use.

In the cross-copolymer according to this embodiment, the residual catalyst components remain in a reduced amount. Accordingly, the cross-copolymer is characterized by having enhanced transparency.

In accordance with the structural parameters of the cross-copolymer, the cross-cross-copolymer exhibits various degrees of transparency. Because of this variation, the standard for transparency after the catalyst component removal may not be defined as one criterion. The present inventors are the first to find out that improved transparency is given to the cross-copolymer, from which the catalyst components have been removed such that the total mass of the residual catalyst aluminum and lithium is 200 ppm or less. Specifically, the total light transmittance is increased by 1% or more when compared with that before the removal of the catalyst components. Also, the haze value can be decreased by 3% or more. Hence, the cross-copolymer according to this embodiment is suitable for applications (e.g., a solar-cell sealant) that need a high degree of transparency.

Meanwhile, in the cross-copolymer according to this embodiment, the residual catalyst components remain in a reduced amount. This cross-copolymer is characterized in that yellowish discoloration caused by addition of a specific antioxidant is suppressed.

Addition of a phenol-based antioxidant to a conventional cross-copolymer unfortunately results in an increase in yellowish discoloration after kneading or a weather resistance test. As used herein, the phenol-based antioxidant refers to an antioxidant having one or more phenol or quinone backbones within a molecule. Examples include various hindered phenol-based antioxidants and BHT.

However, in the cross-copolymer, in which the residual catalyst components remain in a reduced amount, according to the embodiment, the yellowish discoloration after kneading or a weather resistance test can be inhibited even if the phenol-based antioxidant is added. Hence, even if the phenol-based antioxidant is used in the cross-copolymer according to this embodiment, yellowish discoloration can be inhibited, so that the cross-copolymer can be used for solar-cell sealants and/or various skin materials, in particular.

Further, members (e.g., a lamination package, a separator) of a lithium-ion secondary battery, which has an increasing demand recently, need a material in which the content of a lithium ion is reduced. Furthermore, a material substantially free of a lithium ion has been sought. Specific examples include a material in which the content of a lithium ion is 50 ppm or less. In the cross-copolymer according to this embodiment, the residual catalyst components remain in a reduced amount. This cross-copolymer can be suitably used for members of a lithium-ion secondary battery.

### <Cross-copolymer According to This Embodiment>

This embodiment provides a cross-copolymer, wherein the copolymer is produced by carrying out an anionic polymerization in the co-presence of an olefin-(aromatic vinyl compound)-(aromatic polyene) copolymer produced by a coordination polymerization and an aromatic vinyl compound monomer, the cross-copolymer having an olefin-(aromatic vinyl compound)-(aromatic polyene) copolymer chain (sometimes referred to as a main chain) and an aromatic vinyl compound polymer chain (sometimes referred to as a side chain), wherein the total mass of aluminum and lithium, which are catalyst components, contained in the cross-copolymer is 200 ppm or less.

As used herein, examples of the aromatic vinyl compound monomer include, but are not particularly limited to, styrene and various kinds of substituted styrene such as p-methylstyrene, m-methylstyrene, o-methylstyrene, o-t-butylstyrene, m-t-butylstyrene, p-t-butylstyrene, p-chlorostyrene, and o-chlorostyrene. From the industrial aspect, preferred are p-methylstyrene and p-chlorostyrene. Particularly preferred is styrene.

Here, examples of the olefin include, but are not particularly limited to, ethylene and C₃₋₂₀ α-olefins (i.e., propylene, 1-butene, 1-hexene, 4-methyl-1-pentene, 1-octene). As used herein, kinds of the olefin include a cyclic olefin. Examples of the cyclic olefin include vinyl cyclohexane, cyclopentene, and norbornene. In this embodiment, the olefins may be used singly or in combination.

As the olefin used in this embodiment, preferred is ethylene or a mixture of ethylene and the α-olefin (i.e., propylene, 1-butene, 1-hexene, or 1-octene). More preferred is ethylene.

The aromatic polyene used in this embodiment is not particularly limited and has 10 to 30 carbon atoms, and is a monomer having a plurality of double bonds (vinyl groups) and one or more aromatic groups, which allow for a coordination polymerization. In the aromatic polyene, one of the double bonds (vinyl groups) is used for the coordination polymerization. While keeping this polymer status, the remaining double bonds may be used for the anionic polymerization. Preferably, o-divinylbenzene, p-divinylbenzene, and m-divinylbenzene are used singly or in combination.

The most preferable cross-copolymer according to this embodiment is produced by carrying out an anionic polymerization in the co-presence of the ethylene-(aromatic vinyl compound (hereinafter, styrene is specified as a representative compound))-(aromatic polyene (hereinafter, divinylbenzene is specified as a representative compound)) copolymer produced by a coordination polymerization and an aromatic vinyl compound (hereinafter, styrene is specified as a representative compound) monomer, the cross-copolymer having an ethylene-styrene-divinylbenzene copolymer chain (sometimes referred to as a main chain; a soft component) and a polystyrene chain (sometimes referred to as a side chain; a hard component), wherein the total mass of aluminum and lithium, which are catalyst components, contained in the cross-copolymer is 200 ppm or less.

The flexibility of the cross-copolymer, in particular, can be determined by various parameters including: the content of styrene in the ethylene-styrene-divinylbenzene copolymer chain (i.e., the soft polymer chain component (soft segment)); the ratio of the content of the soft component to that of the hard component; The content of the divinylbenzene component which links the soft component chain to the hard component chain; and the molecular fluidity (MFR value) of the whole cross-copolymer, which value can be defined by the molecular weights of the ethylene-styrene-divinylbenzene copolymer chain and the polystyrene chain as well as the content of the divinylbenzene.

Primarily, the storage modulus of the cross-polymer decreases as the content of styrene in the ethylene-styrene-divinylbenzene copolymer chain increases and the crystallinity of the ethylene chain decreases accordingly or as the content of the ethylene-styrene-divinylbenzene copolymer chain (i.e., the soft component) increases.

In view of the above, the flexibility and storage modulus, etc., of the cross-copolymer according to this embodiment can be adjusted by those skilled in the art in accordance with applications thereto in combination with information disclosed in the above references (e.g., WO00/37517, WO2007/139116) regarding cross-copolymers.

Regarding conventional technologies, disclosed are physical properties which are suitable for use of the cross-copolymer and the structures that give the preferable physical properties. Respective publications and pamphlets (e.g., WO2007/139116, WO2013/137326, WO99/45980, JP2001-316431A) describe the physical properties and the structures of, for example, transparent and flexible medical materials including medical tubes, medical bags, sheets, and multilayer sheets. In addition, respective publications (e.g., JP2010-150442A, JP2012-081732A, JP2012-084842A, JP2013-032425A) describe the physical properties and the structures of solar cell sealants and backsheets. The cross-copolymer according to this embodiment can have one of the structures disclosed above.

The cross-copolymer according to this embodiment is not particularly limited as long as the total mass of aluminum and lithium, which are the residual catalyst components, contained in the cross-copolymer is 200 ppm or less. Preferably, the cross-copolymer satisfies all the following conditions (1) to (4) described in WO2013/137326. In this way, the cross-copolymer according to this embodiment can be suitably used for medical tubes.
(1) The cross-copolymer can be produced by a production method comprising polymerization steps including a coordination polymerization step and a subsequent anionic polymerization step. In the coordination polymerization step, a single site coordination polymerization catalyst is used to carry out copolymerization of an ethylene monomer, an aromatic vinyl compound monomer, and an aromatic polyene. Next, an ethylene-(aromatic vinyl compound)-(aromatic polyene) copolymer can be synthesized such that the content of the aromatic vinyl compound unit is from 15 mol% to 30 mol%; the content of the aromatic polyene unit is from 0.01 mol% to 0.2 mol%; and the rest are the content of the ethylene unit. Then, in the anionic polymerization step, an anionic polymerization initiator is used to carry out polymerization in the co-presence of this ethylene-(aromatic vinyl compound)-(aromatic polyene) copolymer and an aromatic vinyl compound monomer.
(2) The weight-average molecular weight of the ethylene-(aromatic vinyl compound)-(aromatic polyene) copolymer obtained in the coordination polymerization step is from 30,000 to 200,000; and the molecular weight distribution (Mw/Mn) is from 1.8 to 4 inclusive.
(3) The total amount (ΔH) of heat of crystal fusion as observed in a temperature range of 0 to 150°C when the cross-copolymer is determined is 25 J/g or less.
(4) The content of the ethylene-(aromatic vinyl compound)-(aromatic polyene) copolymer contained in the cross-copolymer ranges from 70 mass% to 95 mass% inclusive.

The cross-copolymer according to this embodiment is not particularly limited as long as the total mass of aluminum and lithium, which are the residual catalyst components, contained in the cross-copolymer is 200 ppm or less. Preferably, the cross-copolymer satisfies all the following conditions (a) to (e) described in JP2010-150442A. In this way, the cross-copolymer according to this embodiment can be suitably used for solar cell sealants.
(a) In the coordination polymerization step, a single site coordination polymerization catalyst is used to carry out copolymerization of an ethylene monomer, an aromatic vinyl compound monomer, and an aromatic polyene. Next, an ethylene-(aromatic vinyl compound)-(aromatic polyene) copolymer can be synthesized such that the content of the aromatic vinyl compound unit is from 10 mol% to 35 mol%; the content of the aromatic polyene unit is from 0.01 mol% to 0.2 mol%; and the rest are the content of the ethylene unit. Then, in the anionic polymerization step, an anionic polymerization initiator is used to carry out polymerization in the co-presence of a macromonomer of this ethylene-(aromatic vinyl compound)-(aromatic polyene) copolymer and an aromatic vinyl compound monomer.
(b) The weight-average molecular weight of the macromonomer of the ethylene-(aromatic vinyl compound)-(aromatic polyene) copolymer obtained in the coordination polymerization step is from 30,000 to 150,000; and the molecular weight distribution (Mw/Mn) is from 1.8 to 3 inclusive.
(c) The amount (ΔH) of heat of crystal fusion as observed in a temperature range of 0 to 150°C when the macromonomer of this ethylene-(aromatic vinyl compound)-(aromatic polyene) copolymer is determined is 30 J/g or less.
(d) The ratio of TUS (i.e., the total amount of polymerizable unsaturated groups included in a macromonomer; the total number of terminal double bonds + double bonds contained in the divinylbenzene unit) to DOU (i.e., the content of the divinylbenzene unit of the macromonomer) of the macromonomer of this ethylene-(aromatic vinyl compound)-(aromatic polyene) copolymer is within a range: 1.3 ≤ TUS/DOU ≤ 10.
(e) The proportion of mass of the macromonomer of the ethylene-(aromatic vinyl compound)-(aromatic polyene) copolymer in the final cross-copolymer is from 40 mass% to 90 mass% inclusive.

### <Method for Producing Cross-copolymer According to This Embodiment>

A method for producing a cross-copolymer according to this embodiment comprises the steps of:
obtaining a polymer solution containing a cross-copolymer;
preparing liquid containing the polymer solution, water, and an organic acid and subjecting the liquid to emulsification/dispersion, and thereafter;
separating and removing the water from the polymer solution; and
collecting the cross-copolymer from the polymer solution,
wherein the cross-copolymer is produced through a coordination polymerization step of carrying out copolymerization of an olefm monomer, an aromatic vinyl compound monomer and an aromatic polyene using a single-site coordination polymerization catalyst to synthesize an olefin-(aromatic vinyl compound)-(aromatic polyene) copolymer and a subsequent anionic polymerization step of carrying out polymerization in the co-presence of the olefin-(aromatic vinyl compound)-(aromatic polyene) copolymer and an aromatic vinyl compound monomer using an anionic polymerization initiator, and
wherein the organic acid has a pKa of from 1 to 7, and

the solubility of the organic acid is 5 g or more per 100 g of water at 20°C.

Examples of a method for removing a residual catalyst component from a polymer solution include methods disclosed in JP11-335432A and JP06-136034A. In these methods, however, the amount of the residual catalyst component removed from the polymer solution was insufficient. When the method for producing a cross-copolymer according to this embodiment is applied, it is possible to manufacture the cross-copolymer from which the residual catalyst components have been removed in an industrially advantageous manner.

### <Step of Obtaining Polymer Solution Containing Cross-copolymer in Production Method According to This Embodiment>

Examples of the step of obtaining a polymer solution containing a cross-copolymer in the production method according to this embodiment include: obtaining a polymer solution containing a cross-copolymer by carrying out polymerization through the coordination polymerization step and the subsequent anionic polymerization step; and obtaining a polymer solution containing a cross-copolymer after a solvent is added to the cross-copolymer.

A solvent may be added to a cross-copolymer to give a polymer solution containing the cross-copolymer. In this case, the solvent is not particularly limited as long as the cross-copolymer is dissolved in the solvent. Examples of the solvent that can be used include known solvents used for polymerization. Preferred are cyclohexane, methylcyclohexane, toluene, xylene, a C₆₋₁₂ mixed alkane, etc.

### <Coordination Polymerization Step in Production Method According to This Embodiment>

In the coordination polymerization step, a single site coordination polymerization catalyst is used to carry out copolymerization of an olefin monomer, an aromatic vinyl compound monomer, and an aromatic polyene to synthesize an olefin-(aromatic vinyl compound)-(aromatic polyene) copolymer.

Examples of the single site coordination polymerization catalyst used in the coordination polymerization step include, but are not particularly limited to, a catalyst containing a transition metal compound and alumoxane (e.g., methylaluminoxane (or referred to as methyl alumoxane or MAO)), which is a promoter. Such a single site coordination polymerization catalyst is characterized by its high polymerization activity and stability. However, a relatively large amount of the catalyst has to be used. Accordingly, a relatively large amount of a residual catalyst component, in particular, aluminum is included in the final polymer.

Further, the alumoxane used in the coordination polymerization step reacts with an anionic polymerization initiator used in the subsequent anionic polymerization step. Thus, an additional portion of the anionic polymerization initiator that will be consumed during the reaction in the anionic polymerization step has to be added. Thus, a relatively large amount of the anionic polymerization initiator has to be used.

### <Anionic Polymerization Step According to This Embodiment>

In the anionic polymerization step according to this embodiment, an anionic polymerization initiator is used to carry out polymerization in the co-presence of this olefin-(aromatic vinyl compound)-(aromatic polyene) copolymer and an aromatic vinyl compound monomer.

Examples of the anionic polymerization initiator according to this embodiment include, but are not particularly limited to, lithium compounds (e.g., butyl lithium). Generally speaking, when a lithium compound is used as the anionic polymerization initiator, a relatively large amount of lithium is included in the final polymer.

A known method can be used to perform the coordination polymerization step and the anionic polymerization step according to this embodiment. Examples of the polymerization method used include those described in WO00/37517, WO2000/37517, US Patent No. 6559234, WO2007/139116, WO2013/137326, WO99/45980, JP2001-316431A, JP2010-150442A, JP2012-081732A, JP2012-084842A, or JP2013-032425A.

A coordination polymerization step described in WO2013/137326 may be used for the coordination polymerization step according to this embodiment, followed by the anionic polymerization step.

A coordination polymerization step described in JP2010-150442A may be used for the coordination polymerization step according to this embodiment, followed by the anionic polymerization step.

The polymer solution obtained by carrying out polymerization through the coordination polymerization step and the subsequent anionic polymerization step usually contains a solvent and about 10 to 50 mass% of a polymer. Examples of the solvent that can be used include known solvents used for typical polymerization. Preferred are cyclohexane, methylcyclohexane, toluene, xylene, a C₆₋₁₂ mixed alkane, etc.

### <Step of Preparing Liquid Containing the Polymer Solution, Water, and Organic Acid and Subjecting the Liquid to Emulsification/Dispersion>

Conventionally, a polymer solution may be washed with water to remove metal catalyst components such as lithium and aluminum. In this case, an organic solvent and water are subject to phase separation. Accordingly, it has been difficult to increase removal efficiency when a common stirring procedure is used.

In the production method according to this embodiment, however, the organic phase and the water phase are well dispersed. Also, emulsification/dispersion treatment is performed so as to increase the efficiency of removing a catalyst component. Hence, the efficiency of removing the metal catalyst components can be elevated.

In the step of subjecting the liquid to emulsification/dispersion, the emulsification/dispersion is not particularly limited as long as the liquid containing the polymer solution, water, and an organic acid can be subjected to emulsification/dispersion. Here, it is preferable to use an emulsifying disperser. The emulsification/dispersion can be performed with a rotary emulsifying disperser in which the emulsification/dispersion is carried out using a rotor, which rotates at a high speed, and a stator, which engages with the rotor. Examples include a CAVITRON, a product name, (which can be purchased from EuroTec, Inc.) and a Supraton (manufactured by Krupp GmbH., Germany). Any form of the rotor/stator is allowed, and the form may be a sinking comb-type or a hole-type. In addition, examples of the other rotary emulsifying dispersers include rotary, wet micro-pulverizers and rotary homogenizers. Examples of emulsifying dispersers other than the rotary one include homogenizers. Examples of the homogenizers include a high-pressure homogenizer and an ultrasonic homogenizer. Use of the emulsifying disperser increases the emulsification/dispersion treatment efficiency. This emulsification/dispersion treatment is critical to manufacture the cross-copolymer in which the residual catalyst components remain in a reduced amount. This emulsification/dispersion treatment is one of the factors which affect the amount of the residual catalyst components in the cross-copolymer.

In the step of preparing liquid containing the polymer solution, water, and an organic acid and subjecting the liquid to emulsification/dispersion, the polymer solution and water (hereinafter, referred to as washing water) are processed using the above emulsifying disperser. At that time, an organic acid is added. Note that the organic acid may be added beforehand to the washing water or the polymer solution. That is, any liquid containing the polymer solution, water, and an organic acid may be used as long as the liquid contains the polymer solution component, a water component, and an organic acid component. For example, the liquid may contain the polymer solution and an organic acid-containing aqueous solution or the liquid may contain the polymer solution and an organic acid-containing suspension. Either case falls under the liquid containing the polymer solution, water, and an organic acid.

### • Regarding Organic Acid

The organic acid used has a pKa of from 1 to 7. Preferably, the organic acid has a pKa of from 1 to 4. When the organic acid is a polyacid, the pKa as used herein means pKa1 that represents the lowest pKa value.

If the pKa of the organic acid meets the above conditions, the efficiency of removing the catalyst components increases. In addition, even if a specific phenol-based stabilizer is added to the final cross-copolymer, its yellowish discoloration can be inhibited. These matters are advantages.

Further, it is preferable to use an organic acid, the solubility of which is 5 g or more and preferably 10 g or more per 100 g of water at 20°C. If the solubility of the organic acid meets the above conditions, the efficiency of removing the catalyst metals increases. In addition, the amount of the organic acid included in the cross-copolymer is also reduced.

The organic acid is not particularly limited as long as the pKa is from 1 to 7 and the solubility is 5 g or more per 100 g of water at 0°C. Examples of the organic acid include citric acid, tartaric acid, malic acid, acetic acid, lactic acid, aconitic acid, and itaconic acid. Preferred are citric acid and tartaric acid.

The usage of the organic acid is an amount corresponding to a molar equivalent that is 0.5 to 20 times the total molar equivalent of metals included in the polymer solution containing the cross-copolymer. When the usage is less than the amount, the efficiency of removing the catalyst metals decreases. When the usage is higher than the amount, the amount of the organic acid remaining in the polymer increases. This may lower the transparency of the polymer. When the resulting polymer is used for medical purposes, the organic acid eluted from the polymer during a boiling water test may cause a significant change in pH of water.

Preferably, the usage of the organic acid is an amount corresponding to a molar equivalent that is 1 to 5 times the total molar equivalent of metals included in the polymer solution containing the cross-copolymer.

In the production method according to this embodiment, a catalyst-deactivating agent (e.g., water, alcohol, carbon dioxide) may be added beforehand to the resulting polymer solution.

In the step of performing emulsification/dispersion in the production method according to this embodiment, for example, an organic acid may be added to the polymer solution or the washing water. The mixture is processed and washed using an emulsifying disperser.

### <Step of Separating and Removing the Water from The Polymer Solution After Step of Performing Emulsification/Dispersion; and Step of Collecting the Cross-copolymer from the Polymer Solution>

After the step of performing emulsification/dispersion in the production method according to this embodiment, the step of separating and removing the water from the polymer solution and the step of collecting the cross-copolymer from the polymer solution may be carried out to give a cross-copolymer in which the residual catalyst components remain in a reduced amount.

The step of separating and removing the water from the polymer solution is not particularly limited as long as a known procedure can be used to separate and remove the water (washing water) from the polymer solution. For example, the separation and removal procedure includes: separating a mixed solution of a polymer solution and washing water into a polymer solution phase and a washing water phase by means of allowing the mixed solution to stand, heating the mixed solution, and centrifuging the mixed solution, etc. Depending on the degree of removal of the catalyst components, the emulsification/dispersion and the washing water separation may be carried out one or more times. The procedure is preferably repeated twice or more. After the removal of the washing water phase, it is preferable to further likewise process the organic acid-free washing water and polymer solution using an emulsifying disperser to separate a water phase because the catalyst components and the organic acid can be removed further from the polymer solution.

The polymer solution from which the washing water phase has been removed is preferably subject to the step of collecting the cross-copolymer from the polymer solution.

The step of collecting the cross-copolymer from the polymer solution is not particularly limited as long as the cross-copolymer can be recovered from the polymer solution. Examples of the step can include a manipulation and a process (e.g., solvent removal) used for polymer recovery.

A known step may be used as the step of collecting the cross-copolymer from the polymer solution. Examples of the step that can be preferably employed include steam stripping, crumb-forming, degassing using a degassing extruder, and degassing under reduced pressure. Regarding the steam stripping method and the crumb-forming method, methods disclosed in, for example, JP06-136034A and WO2007/139116 are preferably used.

### Examples

Hereinafter, the present invention is illustrated by referring to Experimental Examples. The present invention, however, is not limited by these Experimental Examples.

Source material resins used for Experimental Examples are as follows.

The following cross-copolymers were manufactured by the production methods disclosed in WO2000/37517, WO2007/139116, WO2013/137326, and JP2010-150442A, the full content of which is incorporated herein by citing these publications. The following compositions are likewise determined using the procedures described in the above publications.

These cross-copolymers are each a cross-copolymer obtainable by carrying out an anionic polymerization in the co-presence of a styrene monomer and an ethylene-styrene-divinylbenzene copolymer obtained through a coordination polymerization, the cross-copolymer including an ethylene-styrene-divinylbenzene copolymer chain and a polystyrene chain.

### <Reference Example 1: Synthesis of Polymer Solution Containing Cross-copolymer A>

Here, rac-dimethyl methylenebis(4,5-benzo-1-indenyl)zirconium dichloride was used as a catalyst, and the reaction was carried out as follows.

Polymerization was carried out using a 50-L autoclave equipped with a mixer and a heating and cooling jacket.

Here, 21 kg of methyl cyclohexane (SWACLEAN, manufactured by MARUZEN PETROCHEMICAL CO., LTD.), 3 kg of styrene (manufactured by DENKI KAGAKU KOGYO KABUSHIKI KAISHA), and divinylbenzene (a mixed product containing meth- and para-compound; 70 mmol as divinylbenzene) manufactured by NIPPON STEEL & SUMIKIN CHEMICAL CO., LTD. were placed into the autoclave. The internal temperature was adjusted to 60°C and the mixture was stirred (at 220 rpm). Dry nitrogen gas was made to pass through the liquid for 10 min at a flow rate of 30 L/min for bubbling and water content inside the system and of the polymer solution was purged. Next, 50 mmol of triisobutyl aluminum and 60 mmol of methyl alumoxane (an MMAO-3A/hexane solution, manufactured by Tosoh Akzo Corporation) (designated as MAO in the Tables) in terms of Al were added. Immediately after that, the system was purged with ethylene. After the system was purged sufficiently, the internal temperature was raised to 90°C. Then, ethylene was injected and the system was stabilized at a pressure of 0.34 MPaG (3.4 kg/cm²G). After that, about 50 ml of a toluene solution containing 100 µmol of rac-dimethyl methylenebis(4,5-benzo-1-indenyl)zirconium dichloride and 1 mmol of triisobutyl aluminum was added to the autoclave from a catalyst tank placed on the autoclave. Further, polymerization was performed for 100 min while the internal temperature was kept at 90°C and the pressure at 0.34 MPaG. A small amount (several dozen ml) of the polymer solution was sampled and mixed with methanol to precipitate a polymer. By doing so, a polymer sample during the coordination polymerization step was obtained. From this sample solution, the yield, composition, and molecular weight of the polymer during the coordination polymerization step were determined.

Then, the supply of ethylene to the polymerization vessel was stopped. While the pressure of the ethylene was rapidly released, the internal temperature was cooled to 60°C. Next, 250 mmol of n-butyl lithium was injected, together with the nitrogen gas, from the catalyst tank into the polymerization vessel. Immediately after that, an anionic polymerization started and the internal temperature was raised temporally from 60°C to 75°C. Meanwhile, the temperature was maintained at from 70 to 80°C for 60 min, and the polymerization was continued while stirring (an anionic polymerization step).

The polymerization was ended to give a polymer solution (hereinafter, referred to as polymer solution A). This polymer solution A was used for each Experimental Example.

Note that a small amount (several dozen ml) of the polymer solution A was sampled, air-dried in a draft chamber, and further dried *in vacuo* at 60°C for 8 hours to give a sample of cross-copolymer A. This sample was used to determine the yield, composition, and molecular weight of the cross-copolymer and the amount of residual catalyst components.

The specification of the cross-copolymer obtained in this Reference Example is indicated below, including the content of styrene, the content of divinylbenzene, the weight-average molecular weight (Mw), and the molecular weight distribution (Mw/Mn) of the ethylene-styrene-divinylbenzene copolymer, and the content of the ethylene-styrene-divinylbenzene copolymer, the molecular weight (Mw) of the polystyrene chain, the molecular weight distribution (Mw/Mn), the amount of heat of crystal fusion, as determined by DSC, the content of aluminum, and the content of lithium in the cross-copolymer.

Cross-copolymer A: the content of styrene of the ethylene-styrene-divinylbenzene copolymer was 20 mol%, the content of divinylbenzene was 0.07 mol%, the Mw (weight-average molecular weight) = 121000, the Mw/Mn = 2.5; and the content of the ethylene-styrene-divinylbenzene copolymer was 70 mass%, the Mw of the polystyrene chain = 31000, the Mw/Mn = 1.2, the total amount (ΔH) of heat of crystal fusion, as determined by DSC, was 10J/g or less, the content of aluminum was 750 ppm, and the content of lithium was 400 ppm.

### <Reference Example 2: Cross-copolymer B>

Cross-copolymer B: the content of styrene of the ethylene-styrene-divinylbenzene copolymer was 17 mol%, the content of divinylbenzene was 0.04 mol%, the Mw (weight-average molecular weight) = 91000, the Mw/Mn = 2.2; and the content of the ethylene-styrene-divinylbenzene copolymer was 88 mass%, the Mw of the polystyrene chain = 30000, the Mw/Mn = 1.2, the total amount (ΔH) of heat of crystal fusion, as determined by DSC, was 10 J/g or less, the content of aluminum was 620 ppm, and the content of lithium was 350 ppm.

This is cross-copolymer 1, a source resin, described in WO2013/137326.

### <Preparation of Model Polymer Solution B>

The cross-copolymer B was mixed and dissolved in cyclohexane heated to prepare model polymer solution B containing 20 mass% of the cross-copolymer.

### <Reference Example 3: Cross-copolymer C>

Cross-copolymer C: the content of styrene of the ethylene-styrene-divinylbenzene copolymer was 28 mol%, the content of divinylbenzene was 0.08 mol%, the Mw (weight-average molecular weight) = 90000, the Mw/Mn = 2.4; and the content of the ethylene-styrene-divinylbenzene copolymer was 69 mass%, the Mw of the polystyrene chain = 23000, the Mw/Mn = 1.2, the total amount (ΔH) of heat of crystal fusion, as determined by DSC, was 10 J/g or less, TUS/DOU = 1.7, the content of aluminum was 700 ppm, and the content of lithium was 250 ppm.

This is a cross-copolymer of Production Example 1 described in JP2010-150442A.

### <Preparation of Model Polymer Solution C>

The cross-copolymer C was mixed and dissolved in cyclohexane heated to prepare model polymer solution C containing 20 mass% of the cross-copolymer.

### • How to determine the specification of a cross-copolymer

### <Composition of Copolymer>

First, ¹H-NMR was carried out to determine the content of a styrene unit in a copolymer. An α-500 model (manufactured by JEOL Ltd.) and an AC250 model (manufactured by BRUCKER Inc.) were used as instruments. A sample was dissolved into 1,1,2,2-tetrachloroethane-d2. Measurement was carried out at from 80 to 100°C. TMS (tetramethyl silane) was used as a reference. The area and intensity of peaks (from 6.5 to 7.5 ppm) assigned to a proton of a phenyl group were compared with those of peaks (from 0.8 to 3 ppm) assigned to a proton of an alkyl group.

The proportion of the ethylene-styrene-divinylbenzene copolymer that was obtained in the coordination polymerization step and was included in the cross-copolymer was calculated by comparing the content of styrene in the cross-copolymer and the content of styrene in the ethylene-styrene-divinylbenzene copolymer.

### <Molecular Weight of Copolymer>

Regarding the molecular weight, a GPC (gel permeation chromatography) measurement was used to calculate a number-average molecular weight (Mn) and an weight-average molecular weight (Mw) in terms of a polystyrene standard. The measurement was conducted under the following conditions.
Columns: Two TSK-GEL Multipore HXL-M (ϕ7.8 × 300 mm) (manufactured by Tosoh Corporation) were connected in series and used.
Column temperature: 40°C
Detector: RI, UV light (with a wavelength of 254 nm)
Solvent: THF (tetrahydrofuran)
Liquid flow rate: 1.0 ml/min
Sample concentration: 0.1 mass/volume%
Sample injection volume: 100 µl

Regarding the molecular weight of a polymer insoluble in a THF solvent at room temperature, a high-temperature GPC (gel permeation chromatography) measurement was used to calculate an weight-average molecular weight in terms of a polystyrene standard. An HLC-8121 GPC/HT (manufactured by Tosoh Corporation) and 3 columns (TSKgel GMHHR-H (20) HT, ϕ7.8 × 300 mm) were used and o-dichlorobenzene was used as a solvent to carry out a measurement at 140°C.
Detector: RI
Sample concentration: 0.1 mass/volume%
Sample injection volume: 100 µl
Liquid flow rate: 1.0 ml/min

It is difficult to directly determine the molecular weight of the crossing chain. As used herein, the molecular weight is defined as the same as that of a homopolymer of the aromatic vinyl compound that is not cross-copolymerized. In this way, the molecular weight of a homopolymer of the aromatic vinyl compound as obtained by solvent fractionation is employed.

### <Amount (ΔH) of Heat of Crystal Fusion>

A differential scanning calorimeter "DSC6200 (manufactured by Seiko Instruments Inc.)" was used under a nitrogen air stream to determine the amount of heat of crystal fusion. Specifically, 10 mg of a resin was used. Next, 10 mg of alumina was used as a reference. Then, an aluminum pan was used and a temperature was increased under a nitrogen atmosphere from room temperature to 240°C at a programming rate of 10°C/min, followed by cooling to -120°C at a rate of 20°C/min. After that, a DSC measurement was carried out while the temperature was increased to 240°C at a programming rate of 10°C/min. Finally, the melting point, the amount of heat of crystal fusion, and the glass transition temperature were determined.

### <TUS/DOU>

TUS is defined as the total amount of polymerizable unsaturated groups contained in a macromonomer. Here, TUS represents the total number of double bonds contained in the aromatic polyene (divinylbenzene unit) + terminal double bonds of the polymer. This TUS can be calculated using ¹H-NMR measurement of the macromonomer.

DOU is the content of a divinylbenzene unit of the macromonomer. DOU can be calculated in accordance with US6096849 and/or WO94/10216.

When the TUS/DOU value is large, the content of the aromatic polyene unit was too little. Accordingly, a characteristic as a mixture is enhanced. This results in a loss of the physical properties, in particular, transparency.

In addition, when the TUS/DOU value is small, the content of the aromatic polyene unit is too large. This results in a loss of a function (e.g., flexibility) played by the main chain (macromonomer). Also, the resulting cross-copolymer may have poor molding processability. Besides, gel may be generated in the cross-copolymer.

### <Quantification of Residual Catalyst Components>

The amount of residual catalyst components in the copolymer was quantified using a decomposition method in accordance with RoHS, BS EN1122:2001 (quantification of plastic-cadmium; a wet-decomposition method), and/or RoHS command analysis: IEC62321.

Specifically, 1 g of the sample was heated and decomposed in the presence of sulfuric acid and nitric acid, and quantification was carried out by ICP luminescence analysis (CIROS; SPECTORO Inc.).

### <Total Light Transmittance, Haze>

Regarding the degree of transparency, the total light transmittance and haze of a sheet molded at a thickness of 1 mm by a heating press process were determined using a turbidimeter NDH2000 (manufactured by NIPPON DENSHOKU INDUSTRIES Co., LTD.) in accordance with a method for testing the optical properties of a JIS K-7375 plastic.

### <YI (Yellow Index) Measurement>

YI was measured using a model ZE-2000 (manufactured by NIPPON DENSHOKU INDUSTRIES Co., LTD.) in accordance with JIS K 7105.

### [Experimental Example 1] (Test Using Homogenizer After Acid Was Added)

To 100 ml of the polymer solution A was added 100 ml of an aqueous solution (at a pH of about 2.9) containing 0.19 mass% of citric acid. The mixture was dispersed, using a homogenizer as an emulsifying disperser, at room temperature for a prescribed time.

Note that the equivalent weight of the citric acid in 100 ml of the aqueous solution containing 0.19 mass% of citric acid was 1.53 times the total equivalent weight of lithium and aluminum contained in 100 ml of the polymer solution A.

The homogenizer and conditions used are as follows.
Homogenizer: TK homomixer Mark II model 2.5 (manufactured by PRIMIX Corporation)
Stirring part: a turbine with a diameter of 30 mm and having a communication plate with a diameter of 50 mm
Container used: 300 ml (ϕ 80 × height 100 mm)
Installation position of the stirring part: at a liquid depth of 50 mm
Installation position of the turbine (at a height of 15 mm from the bottom of the container)
Installation position of the communication plate: immediately below the liquid level
The speed of rotation during washing: 8000 rpm
Dispersion time: 3 min

After the dispersion, the solution was allowed to stand and a washing water layer was then separated. As needed, centrifugation was performed to separate a polymer solution layer from the washing water layer. The polymer solution was spread thin on a tray and was air-dried in a draft chamber. Then, the polymer solution was further dried at 60°C for 10 h in a vacuum dryer. After that, a polymer was recovered. The content of each of Li and Al contained in the resulting polymer was quantified. Table 1 shows the results.

### [Experimental Examples 2 to 4]

Table 1 shows the conditions and the results.

In Experimental Example 2, the same test as of Experimental Example 1 was repeated except that citric acid was directly added to the polymer solution A (not to the washing water) and the mixture and water (100 ml) were then subjected to dispersion treatment using a homogenizer.

In Experimental Example 3, the same test as of Experimental Example 1 was repeated except that the dispersion time was changed to 6 min.

In Experimental Example 4, the same test as of Experimental Example 1 was repeated except that the amount of citric acid was changed and an aqueous solution (at a pH of about 2.9) containing 0.57 mass% of citric acid was used as the washing water.

### [Experimental Examples 5 to 8]

Table 1 shows the conditions and the results.

In Experimental Example 5, the same test as of Experimental Example 1 was repeated except that an aqueous solution containing 0.21 mass% of tartaric acid was used instead of the washing water.

In Experimental Example 6, the same test as of Experimental Example 1 was repeated except that an aqueous solution containing 0.42 mass% of tartaric acid was used instead of the washing water.

In Experimental Example 7, the same test as of Experimental Example 2 was repeated except that the organic acid was changed to tartaric acid (0.21 g).

In Experimental Example 8, the same test as of Experimental Example 1 was repeated except that an aqueous solution containing 0.18 mass% of malic acid was used instead of the washing water.

### [Experimental Example 9]

The same test as of Experimental Example 1 was repeated except that the model polymer solution B was used. Table 1 shows the conditions and the results.

### [Experimental Example 10]

The same test as of Experimental Example 1 was repeated except that the model polymer solution C was used. Table 1 shows the conditions and the results.

### [Experimental Example 11]

The cross-copolymer A, as it was, that had been obtained in the Reference Example was used in this Experimental Example. Table 1 shows the results.

### [Experimental Example 12]

The same test as of Experimental Example 1 was repeated except that the organic acid was not added. Table 1 shows the conditions and the results.

### [Experimental Examples 13 to 15]

Table 1 shows the conditions and the results.

In Experimental Example 13, the same test as of Experimental Example 1 was repeated except that a suspension containing 0.4 mass% of benzoic acid was used instead of the washing water.

In Experimental Example 14, the same test as of Experimental Example 2 was repeated except that the organic acid was changed to benzoic acid (0.4 g).

In Experimental Example 15, the same test as of Experimental Example 1 was repeated except that the organic acid was changed to stearic acid (0.78 g).

Because benzoic acid and stearic acid are insoluble in water, they were prepared as a suspension.

Experimental Examples 11 and 12 were compared with Experimental Examples 1 to 8 and 13 to 15. It turns out that catalyst components contained in the polymer remained in a less amount in the Experimental Examples 1 to 8 and 13 to 15. Further, the content of each of the catalyst components was found to be smaller in the cases of Experimental Examples 1 to 8.

**[Table 1]**

| | Polymer solution | Kinds of organic acid | pKa1 | pKa2 | pKa3 | Solubility (at 20°C) (g) in 100 g of water | Concentration of organic acid in washing water mass% | Ratio of organic acid to Li and Al metals (molar equivalent) | Dispersion time (min) | Content (ppm) of Li in a polymer | Content (ppm) of Al in a polymer |
|---|---|---|---|---|---|---|---|---|---|---|---|
| E.Ex. 1 | Polymer solution A | Citric acid | 3.1 | 4.75 | 6.41 | 73 | 0.19 | 1.53 | 3 | 24 | 76 |
| E.Ex. 2 | Polymer solution A | Citric acid | | | | | (0.19 g of organic acid was added to the Polymer solution) | 1.53 | 3 | 16 | 80 |
| E.Ex. 3 | Polymer solution A | Citric acid | | | | | 0.19 | 1.53 | 6 | 20 | 54 |
| E.Ex. 4 | Polymer solution A | Citric acid | | | | | 0.57 | 4.58 | 3 | 11 | 37 |
| E.Ex. 5 | Polymer solution A | Tartaric acid | 3.2 | 4.8 | - | 20.6 | 0.21 | 1.44 | 3 | 35 | 60 |
| E.Ex. 6 | Polymer solution A | Tartaric acid | | | | | 0.42 | 2.88 | 3 | 15 | 33 |
| E.Ex. 7 | Polymer solution A | Tartaric acid | | | | | (0.21g of organic acid was added to the Polymer solution) | 1.44 | 3 | 24 | 60 |
| E. Ex. 8 | Polymer solution A | Malic acid | 3.4 | 5.1 | - | 55.8 | 0.18 | 1.38 | 3 | 48 | 130 |
| E.Ex. 9 | Model Polymer solution B | Citric acid | 3.1 | 4.75 | 6.41 | 73 | 0.19 | 1.53 | 3 | 19 | 65 |
| E.Ex. 10 | Model Polymer solution C | Citric acid | | | | | 0.19 | 1.53 | 3 | 18 | 53 |
| E.Ex. 11 | Cross-copalymer A | Untreated | - | - | - | - | - | - | - | 400 | 750 |
| E.Ex. 12 | Polymer solution A | - (Distilled water) | - | - | - | - | 0 | 0.0 | 3 | 340 | 530 |
| E.Ex. 13 | Polymer solution A | Benzoic acid | 4.2 | - | - | 0.3 | 0.4 | 1.69 | 3 | 300 | 490 |
| E.Ex. 14 | Polymer solution A | Benzoic acid | 4.2 | - | - | 0.3 | (0.40g of organic acid was added to the Polymer solution) | 1.69 | 9 | 160 | 450 |
| E.Ex. 15 | Polymer solution A | Stearic acid | 5 | - | - | Insoluble | 0.78 | 1.44 | 3 | 210 | 290 |

| | | | | | | | | | | | |
|---|---|---|---|---|---|---|---|---|---|---|---|
| **E.Ex.: Experimental Example** | | | | | | | | | | | |

### [Experimental Example 16]

A rotary emulsifying disperser CAVITRON model CD 1010 was used. This disperser includes a rotor, which rotates at a high speed, and a stator, which engages with the rotor, and gives liquid an impact to exert an emulsification/dispersion effect. First, both 5 L of the above polymer solution A and 5 L of the aqueous solution containing 0.19 mass% of citric acid were made to pass through the CAVITRON at a rate of 2 L/min under conditions at a rotation speed of 112000/min and a back pressure of 0.15 MPa. The mixed solution retained in a receiver tank was not subject to phase separation. The mixed solution was then made to pass through the CAVITRON twice more at a rate of 2 L/min under the same conditions (the number of passage through the CAVITRON as designated in Table 2 indicates how many times the above mixed solution passed through the CAVITRON).

The liquid when the number of passage through the CAVITRON was three was allowed to stand in the receiver tank. After an organic phase had been separated from an aqueous phase, the water phase was removed (i.e., this corresponds to removal of a washing water phase by decantation as indicated in Table 2). Thereafter, the organic phase and the three-fold volume of pure water were each made to pass through the CAVITRON at 2 L/min or 6 L/min under the same conditions. While the mixed solution retained in the receiver tank was mixed and was not subject to phase separation, the mixed solution was then made to pass through the CAVITRON twice at a rate of 2 L/min under the same conditions. The liquid when the number of passage through the CAVITRON was three was allowed to stand in the receiver tank. After an organic phase had been separated from an aqueous phase, the water phase was removed (i.e., this corresponds to water washing and decantation as indicated in Table 2). Then, a portion of the organic phase was sampled, air-dried, and degassed *in vacuo* to recover a copolymer. The content of each of Li and Al contained in the resulting polymer was quantified. Table 2 shows the results.

### [Experimental Example 17]

The same process as of Example 11 was repeated except that an aqueous solution containing 0.21 mass% of tartaric acid was used instead of the aqueous solution containing 0.19 mass% of citric acid. Table 2 shows the results.

### [Experimental Example 18]

The same process as of Example 11 was repeated except that distilled water was used instead of the aqueous solution containing 0.19 mass% of citric acid. Table 2 shows the results.

Experimental Example 18 was compared with Experimental Examples 16 and 17. It turns out that the content of each of the catalyst components contained in the polymer was smaller in the Experimental Examples 16 and 17.

**[Table 2]**

| | Kinds of organic acid | Concentration of organic acid mass% | The number of passage through CAVITRON | Removal of washing water phase by decantation | Water washing and decantation | Li content (ppm) | Al content (ppm) |
|---|---|---|---|---|---|---|---|
| E.Ex. 16 | Citric acid | 0.19 | 3 | Once | Once | 8 | 77 |
| E.Ex. 17 | Tartaric acid | 0.21 | 3 | Once | Once | 5 | 41 |
| E.Ex. 18 | Only distilled water | 0 | 3 | Once | Once | 170 | 430 |

| | | | | | | | |
|---|---|---|---|---|---|---|---|
| **E.Ex.: Experimental Example** | | | | | | | |

### [Experimental Example 19]

A Brabender plasticorder (model PL2000, manufactured by Brabender, Inc.) was used. The total of 45 g (100 parts by mass) of a formulation containing the cross-copolymer A (with a content of aluminum of 750 ppm and a content of lithium of 400 ppm) obtained in this Reference Example 1 and the following components (parts by mass) was kneaded at 200°C and 100 rpm for 5 min to prepare a sample. Here, 0.3 part by mass of a photostabilizer LA57 (manufactured by ADEKA Corporation) and 0.1 part by mass of a UV absorber Uvinul 3035 (manufactured by BASF GmbH) were used. Then, 0.1 part by mass of an antioxidant Irganox 1010, manufactured by Chiba Japan KK, was used.

The resulting composition was molded by the above heating press process to produce a sheet with a thickness of 0.5 mm. This sheet was used to determine the transparency (total light transmittance, haze) and the yellowness index (YI) thereof. Table 3 shows the results obtained.

### [Experimental Examples 20 and 21]

Like Experimental Example 19, the cross-copolymer (cross-copolymer A in which the residual catalyst components remained in a reduced amount) as obtained in Experimental Example 16 or 17 was used to produce a composition and a sheet, which were then likewise evaluated. Table 3 shows the conditions and the results.

### [Experimental Example 22]

Like Experimental Example 19, the cross-copolymer (cross-copolymer B in which the residual catalyst components remained in a reduced amount) as obtained in Experimental Example 9 was used to produce a composition and a sheet, which were then likewise evaluated. Table 3 shows the conditions and the results.

### [Experimental Example 23]

Like Experimental Example 19, the cross-copolymer (cross-copolymer C in which the residual catalyst components remained in a reduced amount) as obtained in Experimental Example 10 was used to produce a composition and a sheet, which were then likewise evaluated. Table 3 shows the conditions and the results.

**[Table 3]**

| | Polymer | Haze (%) | Total light transmittance (%) | YI |
|---|---|---|---|---|
| E.Ex. 19 | E.Ex. 11 | 12 | 80 | 8.2 |
| E.Ex. 20 | E.Ex. 16 | 7 | 82 | 5.8 |
| E.Ex. 21 | E.Ex. 17 | 6.5 | 83 | 6.2 |
| E.Ex. 22 | E.Ex. 9 | 6.3 | 84 | 5.3 |
| E.Ex. 23 | E.Ex. 10 | 5.7 | 85 | 5.1 |

| | | | | |
|---|---|---|---|---|
| **E.Ex.: Experimental Example** | | | | |

The sheets of Experimental Examples 20, 21, 22, and 23 had a higher total light transmittance, a lower haze value, and a lower YI than the sheet of Experimental Example 19.

### <Catalyst Extraction Test>

### [Experimental Example 24]

A sheet (with a thickness of 0.5 mm) prepared using the cross-copolymer A of Experimental Example 19 was cut into pieces with a width of about 2 mm and a length of about 6 mm.

Next, 10 g of the pieces of the cross-copolymer and 200 ml of distilled water (at an initial pH of 6.8) were placed in a flask equipped with a reflux device, and the mixture was subjected to extraction for 8 h in a boiling water bath at 100°C. The pH of the resulting extract was measured and the amount of change in pH was determined. In addition, ICP luminescence analysis was conducted to quantify the concentration of each of Li and Al in the extract.

### [Experimental Example 25]

A medical tube (with a diameter of 4 mm) made of a commercially available non-vinyl chloride resin was cut into pieces with a width of about 2 mm. Next, the extraction test was carried out in a manner similar to Comparative Example 7. Then, the amount of change in pH and the concentration of each of Li and Al were determined. In addition, the amounts of the residual catalyst components (Li and Al) in this medical tube were also determined by quantifying the above residual catalyst components. Here, the amount of Li was 20 ppm and the amount of Al was 120 ppm.

### [Experimental Example 26]

The same extraction test as of Experimental Example 24 was repeated except that the sheet (with a thickness of 0.5 mm) of Experimental Example 20 (cross-copolymer A in which the residual catalyst components remained in a reduced amount) was used. Then, the amount of change in pH and the concentration of each of Li and Al were determined.

### [Experimental Example 27]

The same extraction test as of Experimental Example 24 was repeated except that the sheet (with a thickness of 0.5 mm) of Experimental Example 22 was used. Then, the amount of change in pH and the concentration of each of Li and Al were determined.

### [Experimental Example 28]

The same extraction test as of Experimental Example 24 was repeated except that the sheet (with a thickness of 0.5 mm) of Experimental Example 23 was used. Then, the amount of change in pH and the concentration of each of Li and Al were determined.

Table 4 shows the results of Experimental Examples 24 to 28.

**[Table 4]**

| | Change in pH | Concentration (ppm) of Li in extract | Concentration (ppm) of Al in extract |
|---|---|---|---|
| E.Ex. 24 | 1.0 | 0.4 | 1.2 |
| E.Ex. 25 | 0.3 | < 0.1 | < 0.1 |
| E.Ex. 26 | 0.3 | < 0.1 | < 0.1 |
| E.Ex. 27 | 0.3 | < 0.1 | < 0.1 |
| E.Ex. 28 | 0.3 | < 0.1 | < 0.1 |

| | | | |
|---|---|---|---|
| **E.Ex.: Experimental Example** | | | |

The results of Experimental Examples 26, 27, and 28 demonstrated that the sheets of Experimental Examples 20, 22, and 23 had substantially the same amount of change in pH and the same metal concentrations in the extract as of the commercially available medical tube of Experimental Example 25.

That is, the results of Experimental Examples 26, 27, and 28 demonstrate that the cross-copolymers of Experimental Examples 16, 9, and 10 are suitable for medical materials.

### Industrial Applicability

The cross-copolymer in which a residual catalyst component remains in a reduced amount according to the present invention has increased transparency and decreased yellowish discoloration resistance, so that the cross-copolymer is applicable to solar cell sealants. Further, because the residual catalyst components remain in a reduced amount, the cross-copolymer has improved safety, so that the cross-copolymer is suitable for medical resin materials.

## Claims

1. A cross-copolymer which is produced through a coordination polymerization step of carrying out copolymerization of an olefin monomer, an aromatic vinyl compound monomer and an aromatic polyene using a single-site coordination polymerization catalyst to synthesize an olefin-(aromatic vinyl compound)-(aromatic polyene) copolymer and a subsequent anionic polymerization step of carrying out polymerization in a co-presence of the olefin-(aromatic vinyl compound)-(aromatic polyene) copolymer and an aromatic vinyl compound monomer using an anionic polymerization initiator,
wherein a total mass of aluminum and lithium, which are residual catalyst components, contained in the cross-copolymer is 200 ppm or less.

2. The cross-copolymer according to claim 1, wherein all the following conditions (1) to (4) are met:
(1) in the coordination polymerization step, the single site coordination polymerization catalyst is used to carry out the copolymerization of an ethylene monomer, an aromatic vinyl compound monomer, and an aromatic polyene, and then, an ethylene-(aromatic vinyl compound)-(aromatic polyene) copolymer is synthesized such that content of the aromatic vinyl compound unit is from 15 mol% to 30 mol%, content of the aromatic polyene unit is from 0.01 mol% to 0.2 mol%, and the rest are content of the ethylene unit;
(2) an weight-average molecular weight of the ethylene-(aromatic vinyl compound)-(aromatic polyene) copolymer obtained in the coordination polymerization step is from 30,000 to 200,000, and a molecular weight distribution (Mw/Mn) thereof is from 1.8 to 4 inclusive;
(3) a total amount (ΔH) of heat of crystal fusion as observed in a temperature range of 0 to 150°C when the cross-copolymer is determined is 25 J/g or less; and
(4) content of the ethylene-(aromatic vinyl compound)-(aromatic polyene) copolymer contained in the cross-copolymer is from 70 mass% to 95 mass% inclusive.

3. A medical tube comprising the cross-copolymer according to claim 1 or 2.

4. A medical multilayer sheet comprising the cross-copolymer according to claim 1 or 2.

5. The cross-copolymer according to claim 1, wherein all the following conditions (a) to (e) are met:
(a) in the coordination polymerization step, the single site coordination polymerization catalyst is used to carry out the copolymerization of an ethylene monomer, an aromatic vinyl compound monomer, and an aromatic polyene, and then, an ethylene-(aromatic vinyl compound)-(aromatic polyene) copolymer is synthesized such that content of the aromatic vinyl compound unit is from 10 mol% to 35 mol%, content of the aromatic polyene unit is from 0.01 mol% to 0.2 mol%, and the rest are content of the ethylene unit;
(b) an weight-average molecular weight of the ethylene-(aromatic vinyl compound)-(aromatic polyene) copolymer obtained in the coordination polymerization step is from 30,000 to 150,000, and a molecular weight distribution (Mw/Mn) thereof is from 1.8 to 3 inclusive;
(c) an amount (ΔH) of heat of crystal fusion as observed in a temperature range of 0 to 150°C when the ethylene-(aromatic vinyl compound)-(aromatic polyene) copolymer is determined is 30 J/g or less;
(d) a ratio of TUS (i.e., a total amount of polymerizable unsaturated groups included in the copolymer; the total number of terminal double bonds + double bonds contained in the divinylbenzene unit) to DOU (i.e., content of the divinylbenzene unit of the copolymer) of the ethylene-(aromatic vinyl compound)-(aromatic polyene) copolymer is within a range: 1.3 ≤ TUS/DOU ≤ 10; and
(e) content of the ethylene-(aromatic vinyl compound)-(aromatic polyene) copolymer contained in the cross-copolymer is from 40 mass% to 90 mass% inclusive.

6. A solar cell sealant comprising the cross-copolymer according to claim 1 or 5.

7. A method for producing a cross-copolymer, comprising the steps of:
obtaining a polymer solution containing a cross-copolymer;
preparing liquid containing the polymer solution, water, and an organic acid and subjecting the liquid to emulsification/dispersion, and thereafter;
separating and removing the water from the polymer solution; and
collecting the cross-copolymer from the polymer solution,
wherein the cross-copolymer is produced through a coordination polymerization step in which a single site coordination polymerization catalyst is used to carry out copolymerization of an olefin monomer, an aromatic vinyl compound monomer, and an aromatic polyene to synthesize an olefin-(aromatic vinyl compound)-(aromatic polyene) copolymer, and a subsequent anionic polymerization step in which an anionic polymerization initiator is used to carry out polymerization in a co-presence of the ethylene-(aromatic vinyl compound)-(aromatic polyene) copolymer and an aromatic vinyl compound monomer, and
wherein the organic acid has a pKa of from 1 to 7 and
solubility of the organic acid is 5 g or more per 100 g of water at 20°C.

## Patentansprüche

1. Netz-Copolymer, das hergestellt wird durch einen Koordinationspolymerisationsschritt des Durchführens einer Copolymerisation eines Olefinmonomers, eines Monomers einer aromatischen Vinylverbindung und eines aromatischen Polyens unter Verwendung eines Single-Site-Koordinationspolymerisationskatalysators, um ein Olefin-(aromatische Vinylverbindung)-(aromatisches Polyen)-Copolymer zu synthetisieren, und einen nachfolgenden anionischen Polymerisationsschritt des Durchführens einer Polymerisation bei gleichzeitigem Vorhandensein des Olefin-(aromatische Vinylverbindung)-(aromatisches Polyen)-Copolymers und eines Monomers einer aromatischen Vinylverbindung unter Verwendung eines Initiators zur anionischen Polymerisation,
wobei eine in dem Netz-Copolymer enthaltene Gesamtmenge von Aluminium und Lithium, die Restkatalysatorkomponenten sind, 200 ppm oder weniger beträgt.

2. Netz-Copolymer nach Anspruch 1, wobei sämtliche der folgenden Bedingungen (1) bis (4) erfüllt sind:
(1) in dem Koordinationspolymerisationsschritt wird der Single-Site-Koordinationspolymerisationskatalysator verwendet, um die Copolymerisation eines Ethylenmonomers, eines Monomers einer aromatischen Vinylverbindung und eines aromatischen Polyens durchzuführen, und dann wird ein Ethylen-(aromatische Vinylverbindung)-(aromatisches Polyen)-Copolymer so synthetisiert, dass der Gehalt an der Einheit der aromatischen Vinylverbindung von 15 Mol-% bis 30 Mol-% beträgt, der Gehalt an der Einheit des aromatischen Polyens von 0,01 Mol-% bis 0,2 Mol-% beträgt, und der Rest der Gehalt an der Ethyleneinheit ist;
(2) ein Gewichtsmittel des Molekulargewichts des in dem Koordinationspolymerisationsschritt erhaltenen Ethylen-(aromatische Vinylverbindung)-(aromatisches Polyen)-Copolymers beträgt von 30 000 bis 200 000, und eine Molekulargewichtsverteilung (M_{w}/Mₙ) davon beträgt von 1,8 bis einschließlich 4;
(3) ein Gesamtbetrag (ΔH) der Kristallisationswärme, beobachtet in einem Temperaturbereich von 0 bis 150 °C, wenn das Netz-Copolymer untersucht wird, beträgt 25 J/g oder weniger; und
(4) der Gehalt des in dem Netz-Copolymer enthaltenen Ethylen-(aromatische Vinylverbindung)-(aromatisches Polyen)-Copolymers beträgt von 70 Masse-% bis einschließlich 95 Masse-%.

3. Medizinischer Schlauch, umfassend das Netz-Copolymer nach Anspruch 1 oder 2.

4. Medizinische Mehrschichtfolie, umfassend das Netz-Copolymer nach Anspruch 1 oder 2.

5. Netz-Copolymer nach Anspruch 1, wobei all die folgenden Bedingungen (a) bis (e) erfüllt sind:
(a) in dem Koordinationspolymerisationsschritt wird der Single-Site-Koordinationspolymerisationskatalysator verwendet, um die Copolymerisation eines Ethylenmonomers, eines Monomers einer aromatischen Vinylverbindung und eines aromatischen Polyens durchzuführen, und dann wird ein Ethylen-(aromatische Vinylverbindung)-(aromatisches Polyen)-Copolymer so synthetisiert, dass der Gehalt an der Einheit der aromatischen Vinylverbindung von 10 Mol-% bis 35 Mol-% beträgt, der Gehalt an der Einheit des aromatischen Polyens von 0,01 Mol-% bis 0,2 Mol-% beträgt, und der Rest der Gehalt an der Ethyleneinheit ist;
(b) ein Gewichtsmittel des Molekulargewichts des in dem Koordinationspolymerisationsschritt erhaltenen Ethylen-(aromatische Vinylverbindung)-(aromatisches Polyen)-Copolymers beträgt von 30 000 bis 150 000, und eine Molekulargewichtsverteilung (M_{w}/Mₙ) davon beträgt von 1,8 bis einschließlich 3;
(c) ein Betrag (ΔH) der Kristallisationswärme, beobachtet in einem Temperaturbereich von 0 bis 150 °C, wenn das Ethylen-(aromatische Vinylverbindung)-(aromatisches Polyen)-Copolymer untersucht wird, beträgt 30 J/g oder weniger;
(d) ein Verhältnis von TUS (d. h. einer Gesamtmenge von in dem Copolymer enthaltenen polymerisierbaren ungesättigten Gruppen; der Gesamtzahl von endständigen Doppelbindungen + Doppelbindungen, die in der Divinylbenzoleinheit enthalten sind) zu DOU (d. h. Gehalt der Divinylbenzoleinheit des Copolymers) des Ethylen-(aromatische Vinylverbindung)-(aromatisches Polyen)-Copolymers in einem Bereich 1,3 ≤ TUS/DOU ≤ 10 liegt; und
(e) der Gehalt des in dem Netz-Copolymer enthaltenen Ethylen-(aromatische Vinylverbindung)-(aromatisches Polyen)-Copolymers beträgt von 40 Masse-% bis einschließlich 90 Masse-%.

6. Solarzellendichtmaterial, umfassend das Netz-Copolymer nach Anspruch 1 oder 5.

7. Verfahren zur Herstellung eines Netz-Copolymers, umfassend die folgenden Schritte:
Erhalten einer ein Netz-Copolymer enthaltenden Polymerlösung;
Bereiten einer Flüssigkeit, die die Polymerlösung, Wasser und eine organische Säure enthält, und Unterziehen der Flüssigkeit der Emulgierung/Dispergierung; und danach
Abtrennen und Entfernen des Wassers aus der Polymerlösung; und
Sammeln des Netz-Copolymers aus der Polymerlösung,
wobei das Netz-Copolymer hergestellt wird durch einen Koordinationspolymerisationsschritt, in dem ein Single-Site-Koordinationspolymerisationskatalysator verwendet wird, um eine Copolymerisation eines Olefinmonomers, eines Monomers einer aromatischen Vinylverbindung und eines aromatischen Polyens durchzuführen, um ein Olefin-(aromatische Vinylverbindung)-(aromatisches Polyen)-Copolymer zu synthetisieren, und einen nachfolgenden anionischen Polymerisationsschritt, in dem ein Initiator der anionischen Polymerisation verwendet wird, um eine Polymerisation bei gleichzeitigem Vorhandensein des Ethylen-(aromatische Vinylverbindung)-(aromatisches Polyen)-Copolymers und eines Monomers einer aromatischen Vinylverbindung durchzuführen, und
wobei die organische Säure einen pKₐ-Wert von 1 bis 7 aufweist und
die Löslichkeit der organischen Säure bei 20 °C mindestens 5 g pro 100 g Wasser beträgt.

## Revendications

1. Copolymère réticulé qui est produit par une étape de polymérisation coordinative consistant à effectuer la copolymérisation d'un monomère oléfinique, d'un monomère qui est un composé vinylique aromatique et d'un polyène aromatique en utilisant un catalyseur de polymérisation coordinative à un seul site pour synthétiser un copolymère oléfine-(composé vinylique aromatique)-(polyène aromatique), suivie par une étape de polymérisation anionique consistant à effectuer une polymérisation en la co-présence du copolymère oléfine-(composé vinylique aromatique)-(polyène aromatique) et d'un monomère qui est un composé vinylique aromatique en utilisant un amorceur de polymérisation anionique,
où une masse totale d'aluminium et de lithium, qui sont des composants résiduels du catalyseur, contenue dans le copolymère réticulé est de 200 ppm ou moins.

2. Copolymère réticulé selon la revendication 1, où toutes conditions (1) à (4) suivantes sont satisfaites :
(1) à l'étape de polymérisation coordinative, le catalyseur de polymérisation coordinative à un seul site est utilisé pour effectuer la copolymérisation d'un monomère éthylénique, d'un monomère qui est un composé vinylique aromatique et d'un polyène aromatique, puis un copolymère éthylène-(composé vinylique aromatique)-(polyène aromatique) est synthétisé de façon que la teneur en unités composé vinylique aromatique va de 15 % en moles à 30 % en moles, la teneur en unités polyène aromatique va de 0,01 % en moles à 0,2 % en moles et le restant correspond à la teneur en unités éthylène ;
(2) une masse moléculaire moyenne en poids du copolymère éthylène-(composé vinylique aromatique)-(polyène aromatique) obtenu à l'étape de polymérisation coordinative va de 30 000 à 200 000 et une distribution des masses moléculaires (Mw/Mn) de celui-ci va de 1,8 à 4 inclus ;
(3) une quantité totale (ΔH) de chaleur de fusion des cristaux telle qu'observée à l'analyse du copolymère réticulé sur une plage de températures qui va de 0 à 150 °C est de 25 J/g ou moins ; et
(4) une teneur en le copolymère éthylène-(composé vinylique aromatique)-(polyène aromatique) contenu dans le copolymère réticulé va de 70 % en masse à 95 % en masse inclus.

3. Tube médical comprenant le copolymère réticulé selon la revendication 1 ou 2.

4. Feuille multicouche médicale comprenant le copolymère réticulé selon la revendication 1 ou 2.

5. Copolymère réticulé selon la revendication 1, où toutes conditions (1) à (4) suivantes sont satisfaites :
(a) à l'étape de polymérisation coordinative, le catalyseur de polymérisation coordinative à un seul site est utilisé pour effectuer la copolymérisation d'un monomère éthylénique, d'un monomère qui est un composé vinylique aromatique et d'un polyène aromatique, puis un copolymère éthylène-(composé vinylique aromatique)-(polyène aromatique) est synthétisé de façon que la teneur en unités composé vinylique aromatique va de 10 % en moles à 35 % en moles, la teneur en unités polyène aromatique va de 0,01 % en moles à 0,2 % en moles et le restant correspond à la teneur en unités éthylène ;
(b) une masse moléculaire moyenne en poids du copolymère éthylène-(composé vinylique aromatique)-(polyène aromatique) obtenu à l'étape de polymérisation coordinative va de 30 000 à 150 000 et une distribution des masses moléculaires (Mw/Mn) de celui-ci va de 1,8 à 3 inclus ;
(c)une quantité totale (ΔH) de chaleur de fusion des cristaux telle qu'observée à l'analyse du copolymère éthylène-(composé vinylique aromatique)-(polyène aromatique) sur une plage de températures qui va de 0 à 150 °C est de 30 J/g ou moins ; et
(d) un rapport TUS/DOU (où TUS représente une quantité totale de groupements insaturés polymérisables inclus dans le copolymère ; le nombre total de doubles liaisons terminales + de doubles liaisons contenues dans l'unité divinylbenzène ; et DOU représente la teneur en unités divinylbenzène du copolymère) du copolymère éthylène-(composé vinylique aromatique)-(polyène aromatique) est dans la fourchette 1,3 ≤ TUS/DOU ≤ 10 ; et
(e) une teneur en le copolymère éthylène-(composé vinylique aromatique)-(polyène aromatique) contenu dans le copolymère réticulé va de 40 % en masse à 90 % en masse inclus.

6. Matériau d'étanchéité pour photopile comprenant le copolymère réticulé selon la revendication 1 ou 5.

7. Procédé de production d'un copolymère réticulé comprenant les étapes consistant à :
obtenir une solution d'un polymère contenant un copolymère réticulé ;
préparer un liquide contenant la solution du polymère, de l'eau et un acide organique et soumettre le liquide à une émulsification/dispersion puis ;
séparer et éliminer l'eau de la solution du polymère ; et
recueillir le copolymère réticulé à partir de la solution du polymère,
où le copolymère réticulé est produit par une étape de polymérisation coordinative dans laquelle un catalyseur de polymérisation coordinative à un seul site est utilisé pour effectuer la copolymérisation d'un monomère oléfinique, d'un monomère qui est un composé vinylique aromatique et d'un polyène aromatique pour synthétiser un copolymère oléfine-(composé vinylique aromatique)-(polyène aromatique), suivie par une étape de polymérisation anionique dans laquelle un amorceur de polymérisation anionique est utilisé pour effectuer une polymérisation en la co-présence du copolymère oléfine-(composé vinylique aromatique)-(polyène aromatique) et d'un monomère qui est un composé vinylique aromatique et
où le pKa de l'acide organique va de 1 à 7 et
où la solubilité de l'acide organique est de 5 g ou plus par 100 g d'eau à 20 °C.
